(19) Europäisches Patentamt
European Patent Office
Office européen des brevets

(11) **EP 2 703 597 A1**

(12) **EUROPEAN PATENT APPLICATION**

(43) Date of publication:
**05.03.2014 Bulletin 2014/10**

(51) Int Cl.:
**E21B 21/06** *(2006.01)*    **G01N 33/28** *(2006.01)*

(21) Application number: **12306047.7**

(22) Date of filing: **03.09.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Geoservices Equipements**
**95700 Roissy en France (FR)**

(72) Inventors:
• **Guerriero, Nicolas**
**95971 Roissy en France (FR)**

• **Lessi, Jacques**
**95971 Roissy en France (FR)**

(74) Representative: **Rzaniak, Martin**
**Schlumberger**
**1, rue Henri Becquerel**
**B.P. 202**
**92142 Clamart (FR)**

Remarks:
Amended claims in accordance with Rule 137(2) EPC.

(54) **Method of calibration for the use in a process of determining the content of a plurality of compounds contained in a drilling fluid**

(57)    Method of calibration intended to be used in a process of determining the content of a plurality of compounds contained in a drilling fluid, the method comprising obtaining a calibration mud (302); performing a gas extraction from said calibration mud with at least two extraction stages in order to obtain at least two extracted gases; measuring parameters representative of the quantities of said calibration compounds in said extracted gases; and calculating correction factors using said measured parameters, the correction factors being intended to be used in said process.

The calibration mud is an artificial mud comprising a sample (304) of a drilling fluid mixed with calibration compounds (306) comprising at least two, preferably four, different hydrocarbon compounds, each of said hydrocarbon compounds having at least 5 carbon atoms.

Process of determining the content of a plurality of compounds contained in a drilling fluid implementing said method of calibration.

Method of preparation (300) of said calibration mud, comprising the following steps: obtaining (A) the initial mud sample from a drilling fluid; obtaining said calibration compounds and mixing (B) them in order to obtain a calibration mixture (308); adding (C) said calibration mixture into said initial mud sample in order to obtain an emulsified mud (310); and obtaining (D) the calibration mud from the emulsified mud.

FIG.6

**Description**

The present invention relates to a method of calibration of the type intended to be used in a process of determining the content of a plurality of compounds contained in a drilling fluid, the method comprising:

- obtaining a calibration mud containing calibration compounds;
- performing a gas extraction from said calibration mud with at least two extraction stages in order to obtain at least two extracted gases containing said calibration compounds;
- measuring parameters representative of the quantities of said calibration compounds in said extracted gases; and
- calculating correction factors using said measured parameters, the correction factors being intended to be used in said process in order to calculate said content.

The present invention also relates to a process of determining the content of a plurality of compounds contained in a drilling fluid, the process involving said method of calibration.

The present invention further relates to a method of preparation of the calibration mud.

During the drilling of a petroleum or gas well, it is known how to perform an analysis of the gas compounds contained in the drilling fluid emerging from the well, this fluid being commonly designated as "drilling mud".

This analysis gives the possibility of reconstructing the geological succession of the crossed formations during the drilling and is involved in the determination of the possibilities of exploiting encountered fluid deposits.

This analysis performed continuously comprises two main phases. A first phase consists of continuously sampling the drilling mud in circulation, and then of bringing it into an extraction enclosure where a certain number of compounds carried by the mud (for example hydrocarbon compounds, carbon dioxide, hydrogen sulfide, helium and nitrogen) are extracted from the mud as a gas.

A second phase consists of transporting the extracted gases towards an analyzer where these gases are described and in certain cases quantified.

For extracting the gases from the mud, a degasser with mechanical stirring of the type described in FR 2 799 790 is frequently used.

The gases extracted from the mud, mixed with a carrier gas introduced into the degasser are conveyed by suction through a gas extraction conduit up to an analyzer which allows quantification of the extracted gases.

With such a device it is possible to significantly and specifically extract the very volatile gases present in the mud, for example $C_1$-$C_5$ hydrocarbons, notably when it is used with a device for heating the drilling mud, placed upstream from the degasser or in the latter.

However the extraction, in the degasser, of the compounds contained in the mud is not total and the extraction efficiency, defined as the amount of an extracted compound referred to the total amount of this same compound initially contained in the mud, depends on the nature of the compound. It is therefore known how to empirically correct the measurement carried on the gas fraction extracted for each compound with a correction factor depending on the compound in order to provide an estimate of the actual content of the compound in the drilling mud.

This is notably the case in muds based on oils or synthetic products, in which the hydrocarbons are relatively soluble.

In order to improve the accuracy of the measurement, EP-A-1 710 575 describes a method of the aforementioned type wherein a same calibration sample of the drilling fluid, containing the different compounds to be extracted, successively undergoes several extraction stages in the degasser, the amount of extracted gas being measured at each extraction stage.

On the basis of the gas fractions measured at each extraction stage for each compound, a correction factor relating the content of a given compound to the measured fraction during a first extraction stage in the degasser may be determined experimentally for each compound.

With such a method the accuracy of the measurement may be considerably improved. In order to apply it, it is necessary to have the calibration sample pass at least twice in the degasser and to analyze the gas composition of the extracted gases of each compound to be analyzed, which requires having available an initial mud sample containing a large amount of compounds, the intention being to evaluate the extraction efficiency thereof. Accordingly, the results in certain cases may not be very accurate, notably for heavy compounds which are difficult to extract from the drilling mud.

EP-A-2 380 017 aims at further improving the accuracy of the determination of the content of a plurality of compounds contained in a drilling fluid. It describes a method comprising the step of calculating, for at least each second compound of a second group of compounds, the content of said second compound in the drilling fluid on the basis of the representative information measured for the second compound under second given extraction conditions, advantageously identical with the first given extraction conditions, and a second correction factor calculated from a calculation equation relating the second correction factor to a plurality of parameters independent of the second compound and of the given extraction conditions and to a thermodynamic factor characteristic of the second compound which depends on at least one thermodynamic parameter representative of the second compound, the independent parameters being determined from each first correction factor and from the calculation equation.

However, all of the above methods require using a calibration mud. The calibration mud is a sample taken from the drilling fluid itself after having crossed geological formations containing hydrocarbons. As a result, the hydrocarbons content in the calibration mud depends on the crossed formations. The calibration method cannot be carried out at any time and the hydrocarbons content in the calibration mud may not be sufficient and/or adapted to perform an accurate calibration.

One aim of the invention is therefore to provide a method of calibration which can be performed at any time, advantageously on an oil rig, and which enables accurate calibration.

To that end, the invention relates to a method of calibration of the above type, wherein the calibration mud is an artificial mud sample comprising a sample of a drilling fluid mixed with the calibration compounds, the calibration compounds comprising at least two, preferably four, different hydrocarbon compounds being in part in liquid state at operative pressure, temperature and volume conditions.

The method according to the invention may comprise one or more of the following features, taken individually or according to any technically possible combination(s):

- the operative pressure, temperature and volume conditions are atmospheric conditions, and said hydrocarbon compounds having at least 5 carbon atoms;
- each of said hydrocarbon compounds has between 5 and 7 carbon atoms;
- each of said hydrocarbon compounds has 5 or 6 carbon atoms;
- said calibration compounds are normal pentane, isopentane, normal hexane and 2,2-dimethylpentane
- each of said calibration compounds has a respective concentration in the calibration mud, said concentrations of the calibration compounds being between 0.01 and 1 g/l of mud;
- said calibration mud is substantially free of surfactant.

The invention also relates to a process of determining the content of a plurality of compounds contained in a drilling fluid comprising:

- implementing the above described method of calibration; and
- obtaining at least one second sample of the drilling fluid;
- performing a gas extraction from said second sample in order to obtain at least a third extracted gas containing said plurality of compounds;
- measuring second parameters representative of the quantities of said plurality of compounds in said third extracted gas; and
- calculating said content using said second parameters and said correction factors.

The process according to the invention may comprise the following features: the step of obtaining at least one second sample of the drilling fluid is iterated.

By "iterated", it is meant that the step is repeated one or several times. For example it is performed on a regular basis in order to analyze the drilling fluid on a regular basis.

The invention also relates to a method of preparation of a calibration mud intended to be used in the above described method of calibration, the method of preparation comprising the following steps advantageously performed on a rig:

- obtaining an initial mud sample from a drilling fluid;
- obtaining said calibration compounds and mixing them in order to obtain a calibration mixture;
- adding said calibration mixture into said initial mud sample in order to obtain an emulsified mud; and
- obtaining the calibration mud from the emulsified mud.

The method of preparation may comprise one or more of the following features, taken individually or according to any technically possible combination(s):

- the step of adding said calibration mixture into said initial mud sample includes an emulsification of the calibration mixture and at least a fraction of the initial mud sample;
- said emulsification is carried out using at least one ultrasonic probe in order to send an ultrasonic signal into said fraction of the initial mud sample;
- the step of adding said calibration mixture into said initial mud sample comprises the following substeps: extracting mud from the initial mud sample in order to obtain a sub-sample of mud and a remaining part of mud; emulsification of the calibration mixture and the sub-sample of mud in order to obtain an emulsified sub-sample; and mixing the emulsified sub-sample and the remaining part of mud in order to obtain the emulsified mud;

- said ultrasonic signal corresponds to a power comprised between 0.5 and 2.0 W/cm$^3$ of said sub-sample, preferably between 0.75 and 1.5 W/cm$^3$ of said sub-sample;
- said ultrasonic signal has a frequency ranging from 18000 Hz to 25000 Hz, preferably about 20000 Hz;
- said step of obtaining the calibration mud includes homogenizing said emulsified mud in order to obtain said calibration mud.

The invention will be better understood upon reading the description which follows, given only as an example, and made with reference to the appended drawings, wherein:

Fig. 1 is a schematic vertical sectional view of a drilling installation;
Fig. 2 is a schematic vertical sectional view analogous to Fig. 1 of a calibration assembly intended to apply a method of calibration according to the invention;
Fig. 3 is a curve illustrating the contents of different gas fractions extracted from a calibration mud during successive passages of the calibration mud in the calibration stage of Fig. 2;
Fig. 4 is a curve illustrating the different correction factors calculated in a process of determining the content of a plurality of compounds in the drilling fluid according to the invention versus the thermodynamic factor characteristic of each compound;
Fig. 5 is a view analogous to Fig. 4 illustrating a second exemplary application of the method according to the invention; and
Fig. 6 is a diagram of a method of preparation of a calibration mud according to the invention.

In all the following, the terms of "upstream" and "downstream" are understood relatively to the normal direction of circulation of a fluid in a conduit.

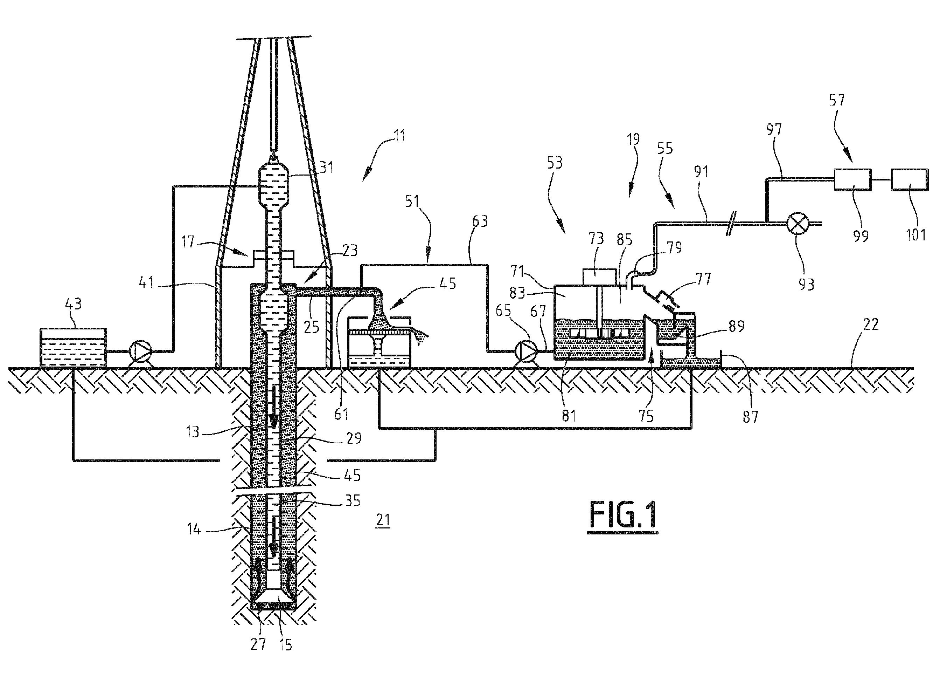

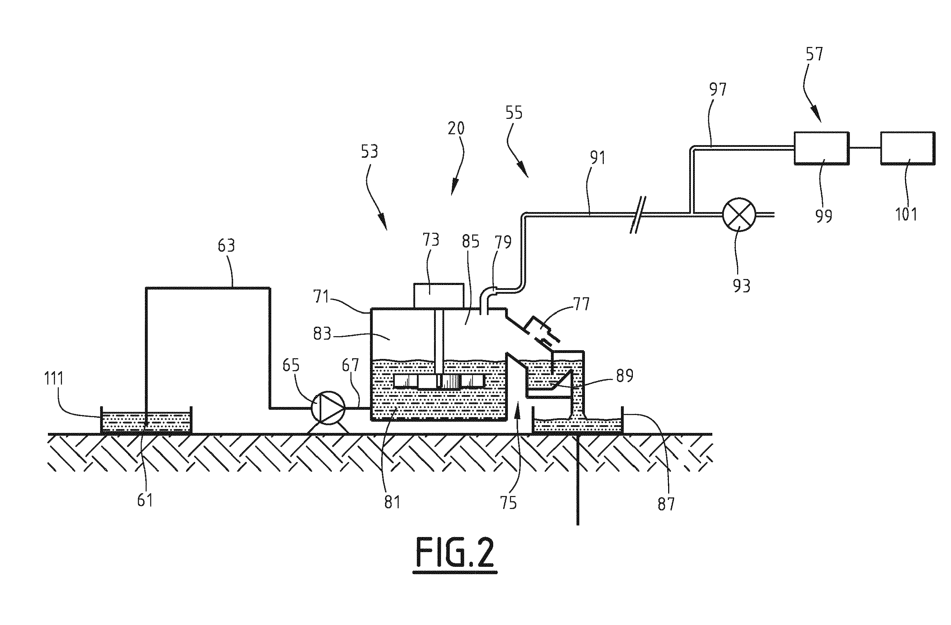

A first determination method according to the invention is intended to be applied in a drilling installation 11 of a well for producing fluid, notably hydrocarbons, such as an oil well. Such an installation 11 is illustrated by Figs. 1 and 2.

This installation 11 comprises a drilling conduit 13 positioned in a cavity 14 pierced by a rotary drilling tool 15, a surface installation 17, and an assembly 19 for analyzing the gases contained in the drilling fluid.

The installation 11 further comprises a calibration assembly 20 illustrated in Fig. 2.

With reference to Fig. 1, the drilling conduit 13 is positioned in the cavity 14 pierced in the subsoil 21 by the rotary drilling tool 15. It extends in an upper portion of the height of the cavity 14 which it delimits. The cavity 14 further has a lower portion directly delimited by the subsoil.

The drilling conduit 13 includes at the surface 22 a well head 23 provided with a conduit 25 for circulation of the fluid.

The drilling tool 15 comprises, from bottom to top in Fig. 1, a drilling head 27, a drill string 29, and a head 31 for injecting drilling fluid. The drilling tool 15, is driven into rotation by the surface installation 17.

The drilling head 27 comprises means 33 for piercing the rocks of the subsoil 21. It is mounted on the lower portion of the drill string 29 and is positioned in the bottom of the cavity 14.

The string 29 comprises a set of hollow drilling tubes. These tubes delimit an inner space 35 which allows the drilling fluid injected through the head 31 from the surface 22 to be brought as far as the drilling head 27. For this purpose, the injection head 31 is screwed onto the upper portion of the drill string 29.

This drilling fluid, commonly designated with the term of « drilling mud », is essentially liquid.

The surface installation 17 comprises means 41 for supporting and driving into rotation the drilling tool 15, means 43 for injecting the drilling fluid and a vibrating sieve 45.

The injection means 43 are hydraulically connected to the injection head 31 for introducing and circulating the drilling fluid in the internal space 35 of the drill string 29.

The drilling fluid is introduced into the inner space 35 of the drill string 29 through the injection means 43. This fluid flows downwards down to the drilling head 27 and passes into the drilling conduit 13 through the drilling head 27. This fluid cools and lubricates the piercing means 33. The fluid collects the solid debris resulting from the drilling and flows upwards through the annular space defined between the drill string 29 and the walls of the drilling conduit 13, and is then discharged through the circulation conduit 25.

The inner space 35 opens out facing the drilling head 27 so that the drilling fluid lubricates the piercing means 33 and flows upwards in the cavity 14 along the conduit 13 up to the well head 23, while discharging the collected solid drilling debris, in the annular space 45 defined between the string 29 and the conduit 13.

The drilling fluid present in the cavity 14 maintains hydrostatic pressure in the cavity, which prevents breakage of the walls delimiting the cavity 14 not covered by the conduit 13 and which further avoids eruptive release of hydrocarbons in the cavity 14.

The circulation conduit 25 is hydraulically connected to the cavity 14, through the well head 23 in order to collect the drilling fluid from the cavity 14. It is for example formed by an open return line or by a closed tubular conduit.

In the example illustrated in Fig. 1, the conduit 25 is a closed tubular conduit.

The vibrating sieve 45 collects the fluid loaded with drilling residues which flow out of the circulation conduit 25, and separates the liquid from the solid drilling residues.

The analysis assembly 19 comprises a device 51 for sampling drilling fluid in the conduit 25, a device 53 for extracting a gas fraction of the compounds contained in the drilling fluid, a device 55 for transporting gas fractions and an analysis device 57.

The sampling device 51 comprises a sampling head 61 immersed in the circulation conduit 25, a sampling conduit 63 connected upstream to the sampling head 61, a pump 65 connected downstream to the sampling conduit 63, and a conduit 67 for bringing the drilling fluid into the extraction device 53, connected to an outlet of the pump 65.

The sampling device 51 is further advantageously provided with an assembly for heating the sampled fluid (not shown). This heating assembly is for example positioned between the pump 65 and the extraction means 53 on the supply conduit 67.

The pump 65 is for example a peristaltic pump capable of conveying the drilling fluid sampled by the head 61 towards the extraction means 53 with a determined fluid volume flow rate $Q_m$.

The extraction device 53 comprises an enclosure 71 into which the supply conduit 67 opens out, a rotary stirrer 73 mounted in the enclosure 71, a mud discharge conduit 75, an inlet 77 for injecting a carrier gas and an outlet 79 for sampling the extracted gas fractions in the enclosure 71.

The enclosure 71 has an inner volume for example comprised between 0.04L and 3 L. It defines a lower portion 81 of average volume $V_m$, kept constant, in which circulates the drilling fluid stemming from the supply conduit 67 and an upper portion 83 of average volume $V_g$ kept constant and defining a gas head space above the drilling fluid.

The mud supply conduit 67 opens out into the lower portion 81.

The stirrer 73 is immersed into the drilling fluid present in the lower portion 81. It is capable of vigorously stirring the drilling fluid in order to extract the extracted gases therefrom.

The discharge conduit 75 extends between an overflow passage 85 made in the upper portion 83 of the enclosure 71 and a retention tank 87 intended to receive the drilling fluid discharged out of the extraction device 53.

The discharge conduit 75 is advantageously bent in order to form a siphon 89 opening out facing the retention tank 87 above the level of liquid contained in this tank 87.

Alternatively, the drilling fluid from the conduit 75 is discharged into the circulation conduit 25.

In this example, the inlet for injecting a carrier gas 77 opens out into the discharge conduit 75 upstream from the siphon 89 in the vicinity of the overflow passage 85.

Alternatively, the inlet 77 opens out into the upper portion 83 of the enclosure 71.

The sampling outlet 79 opens out into an upper wall delimiting the upper portion 83 of the enclosure 71.

The drilling fluid introduced into the enclosure 71 via the supply conduit 67 is discharged by overflow into the discharge conduit 75 through the overflow passage 85. A portion of the discharged fluid temporarily lies in the siphon 89 which prevents gases from entering the upper portion 83 of the enclosure 71 through the discharge conduit 75.

The introduction of gas into the enclosure 71 is therefore exclusively carried out through the inlet for injecting a carrier gas 77.

In the example illustrated by Fig. 1, the carrier gas introduced through the introduction inlet 77 is formed by the surrounding air around the installation, at atmospheric pressure. Alternatively, this carrier gas is another gas such as nitrogen or helium.

The transport device 55 comprises a line 91 for transporting the extracted gases towards the analysis device 57 and suction means 93 for conveying the gases extracted out of the enclosure 71 through the transport line 91.

The transport line 91 extends between the sampling outlet 79 and the analysis device 57. It advantageously has a length comprised between 10 m and 500 m, in order to move the analysis device 57 away from the well head 23 into a non-explosive area.

The transport line 91 is advantageously made on the basis of a metal or polymer material, notably polyethylene and/or polytetrafluoroethylene (PTFE).

The analysis device 57 comprises a sampling conduit 97 tapped on the transport line 91 upstream from the suction means 93, an instrumentation 99, and a computing unit 101.

The instrumentation 99 is capable of detecting and quantifying the gas fractions extracted out of the drilling fluid in the enclosure 71 which have been transported through the transport line 91.

This instrumentation for example comprises infrared detection apparatuses for the amount of carbon dioxide, chromatographs with flame ionisation detectors (FID) for detecting hydrocarbons or further with thermal conductivity detectors (TCD) depending on the gases to be analyzed.

It may also comprise a chromatography system coupled with a mass spectrometer, this system being designated by the acronym "GC-MS". It may comprise an isotope analysis apparatus as described in Application EP-A-1 887 343 of the Applicant.

Online simultaneous detection and quantification of a plurality of compounds contained in the fluid, without any manual sampling by an operator, is therefore possible within time intervals of less than 1 minute.

As this will be seen below, the computing unit 101 is capable of calculating the content of a plurality of compounds to be analyzed present in the drilling fluid on the basis of the value of the extracted gas fractions in the enclosure 71, as determined by the instrumentation 99, and on the basis of correction factors p(i) specific to each compound to be analyzed.

The calibration assembly 20 illustrated in Fig. 2 is in this example formed by the sampling device 51, the extraction device 53, the transport device 55 and the analysis device 57 of the analysis assembly 19.

However, this calibration assembly 20 further comprises an upstream tank 111 intended to receive a calibration sample of the drilling fluid with view to having this sample pass several successive times in the extraction device 53 in order to be subject to several extraction stages therein.

In one alternative, at least the extraction device of the calibration assembly 20 is distinct from the extraction device 53 of the analysis assembly 19.

In this case, the extraction devices of the analysis assembly 19 and of the calibration assembly 20 are substantially identical and for example have an enclosure geometry 71 which is identical (notably in size or in volume), and an identical stirrer 73.

Thus, the extraction of the gas fractions from the calibration sample contained in the upstream tank 111 may be carried out under the same extraction conditions as the extraction of the gas fractions in a drilling fluid sample continuously taken in the drilling conduit 25 during the analysis of this fluid.

This notably implies that the temperature of the drilling fluid in the enclosure 71, the pressure P of the gas head space located above the fluid present in the enclosure 71, the drilling fluid flow rate $Q_m$ admitted into the enclosure 71, and the sampled gas flow rate $Q_g$, the volume $V_m$ of drilling fluid in the enclosure 71, and the gas volume Vg present in the enclosure 71, the nature of the stirring as well as the stirring rate, are substantially identical in the extraction devices of the calibration assembly 20 and of the analysis assembly 19.

The drilling fluid for example is formed by mud with water or mud with oil. In general, drilling mud compounds contain hydrocarbons with $C_n$ n<20. Today, hydrcarbons that we want to be analysed are up to $C_8$, however higher $C_n$ can be analysed if wanted..

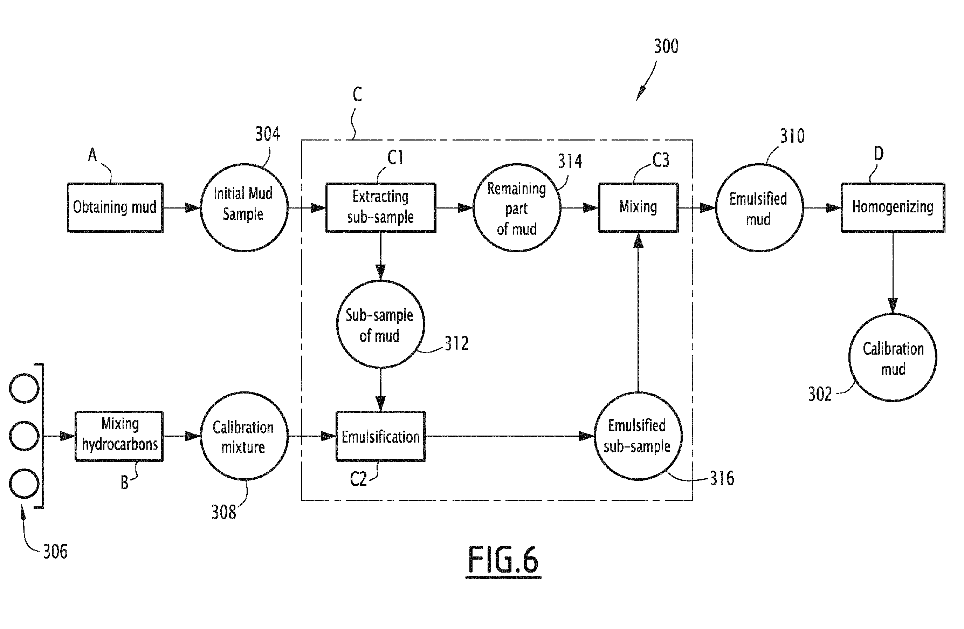

A method 300 of preparation of a calibration mud 302 will now be described with reference to Figure 6.

The calibration mud 302 is intended to be used in a calibration method, usually part of a process of determining the content of the compounds to be analyzed.

The method 300 comprises a step A of obtaining an initial mud sample 304 from the drilling fluid; a step B of obtaining calibration compounds 306 and mixing the calibration compounds 306 in order to obtain a calibration mixture 308; a step C of adding said calibration mixture 308 into said initial mud sample 304 in order to obtain an emulsified mud 310 made with ultrasonic mixer or the like; and a step D of obtaining the calibration mud 302 from the emulsified mud 310. The mixer will advantageously be an ultrasonic mixer. However, other type of mixer may be used realizing the same effect: creating an emulsion without the use of surfactant. If an ultrasonic mixer is used, the emulsion will be created by cavitation effect.

Steps A, B, C and D are advantageously carried out on well site. The drilling installation is represented in Fig. 1 and 2.

The initial mud sample 304 advantageously has a volume of 5 to 50 litres, for example about 20 litres. The initial mud sample 304 is preferably kept in a sealable bucket.

The initial mud sample 304 is for example recovered from an active pit in which the drilling fluid is prepared.

In one embodiment, the calibration compounds 306 comprise different hydrocarbons compounds, in particular alkanes being in liquid state under atmospheric conditions. It would be possible by modifying the PVT conditions to use other alkanes at liquid state or close to liquid state. For example, hydrocarbons compounds having 3 or 4 carbon atoms could be used as well as compounds having between 8 and 10 carbon atoms.

Under atmospheric conditions, each of said hydrocarbons compounds has at least 5 carbon atoms, advantageously between 5 and 7 carbon atoms, for example 5 or 6 carbon atoms. As will be seen below, a good resolution is obtained when the analytical cycle time is the shortest, i. e. with hydrocarbon compounds having between 5 and 6 carbon atoms.

In a particular embodiment, said calibration compounds 306 are normal pentane, isopentane, normal hexane and 2,2- or 2,3-dimethylbutane.

Step C advantageously includes emulsifying the calibration mixture 308 and at least a fraction 312 of the initial mud sample 304. Using only a fraction of the initial mud sample 304 facilitates the emulsification. Advantageously the volume of said fraction 312 is less than 1 litre, and more generally less than 10% [check] of the volume of the initial mud sample 304.

In a particular embodiment, step C includes a substep C1 of extracting a sub-sample from the initial mud sample 304 in order to obtain a sub-sample of mud 312 and a remaining part of mud 314; a substep C2 of emulsification of the sub-sample of mud 312 with the calibration mixture 308 in order to obtain an emulsified sub-sample of mud 316; and a substep C3 of mixing the remaining part of mud 314 and the emulsified sub-sample of mud 316 in order to obtain the emulsified mud 310.

In substep C1, the initial mud sample 304 is for example directly separated into the sub-sample of mud 312 and the remaining part of mud 314.

Said emulsification C2 is for example carried out by mixing the sub-sample of mud 312 and the calibration mixture 308, while using at least one ultrasonic probe to disperse the calibration mixture into the sub-sample by cavitation effect.

Said ultrasonic signal advantageously corresponds to a power comprised between 0.5 and 2 W/cm$^3$ of said sub-sample 312, preferably between 0.75 and 1.5 W/cm$^3$ of said sub-sample 312.

For example said ultrasonic signal has a frequency ranging from 18000 Hz to 25000 Hz, preferably a frequency of about 20000 Hz.

Substep C2 advantageously includes homogenizing the emulsified sub-sample 316, in order to improve the stability of the emulsified sub-sample 316.

The step D of obtaining the calibration mud 302 advantageously includes homogenizing said emulsified mud 310, in order to improve the stability of the calibration mud 302.

Advantageously, each of said calibration compounds 306 has a respective concentration in the calibration mud 302, said concentrations of the calibration compounds 306 being between 0.01 and 1 g/l of mud, for example between 0.02 and 0.2 g/l of mud..

The ratio of C5 compounds over C6 compounds, in ppm/ppm, in the calibration mud 302 is advantageously close to 2. To that end, the ratio, in ppm/ppm, of C5 compounds over C6 compounds in the calibration mixture 308 is advantageously set at about 2.

According to a particular embodiment, said calibration mud 302 is substantially free of surfactant. This means that no surfactant is added in the calibration mixture 308, nor at a later stage during steps C and D. This enables to enhance the extraction efficiency of the calibration compounds.

By using no surfactant the calibration method is non-invasive, as no other compound is added to the drilling fluid which would affect the extraction efficiency.

Thanks to the method of preparation described above, a calibration mud 302 can easily be obtained at any time and is representative of a drilling fluid having crossed formations containing hydrocarbons. As a consequence, the method of calibration can be performed at any time, advantageously on an oil rig, and enables accurate calibration.

Thanks to using hydrocarbon compounds having at least 5 carbon atoms, the calibration mixture 308 is liquid, which facilitates step C.

Using C5 and C6 hydrocarbon compounds in the calibration mud 302 allows reducing the time needed for calibration, as heavier hydrocarbon compounds require a longer analysis time. We may use C7 to C9 hydrocarbons. But if those heavier hydrocarbons are used, a longer analysis time would be required. Therefore it is preferred to use C5 and C6 hydrocarbons.

For other reason, we may want to consider using C7 or C8 if we are interested in less margin error on the correction factors. For example, we may use C7 for Water Based Mud even if it requires a longer analysis time, because emulsions with Water Based Mud are less stable and C7 will provide a better reliability.

Example of preparation:

The method 300 may be carried out using the following material:

- an ultrasonic probe, at 20 kHz, with a maximum power of 750W,
- a magnetic agitator,
- a 600ml beaker,
- a 1 litre beaker,
- a sample bottle of calibration mixture,
- a 2.5 ml syringe,
- a bucket of 25 litres,
- a convenient tool for manual stirring.

Step A: to obtain the initial mud sample, collect 20 litres of drilling fluid from the active pit, once it has been prepared by the mud engineer. Keep the initial mud sample in a sealable bucket.

Step B: a blend of calibration compounds is packaged in a 3 cc sample bottle. The blend comprises C5+ alkanes.

Step C1: To extract the sub-sample of mud, pour 450 ml of the initial mud sample in a beaker dedicated to the preparation, for example a beaker of 600 cc, adapted to the ultrasonic probe's shape.

Step C2: add a magnetic stirring bar in the beaker. Put the beaker in a larger one containing fresh water to limit mud's heating during the emulsification. Set the ultrasonic probe at about 60% of amplitude (400 W) and timing to 10 minutes. Place the probe deep into the beaker, more or less at the centre of the volume. Prepare a syringe of 2.5 cc. Fill the syringe with for example 2.5 cc for injection of the calibration mixture. Start the ultrasonic probe and inject the syringe content into the beaker. Injection can be slow and take up to 30 seconds. Wait until the sonification stops. Cover the beaker with a plastic film and use a magnetic stirrer for 10 minutes in order to improve the emulsified sub-sample stability.

Step C3: pour softly the content of the beaker into the bucket of mud.

Step D: homogenize the 20 litres of emulsion for 1 to 2 minutes, using the most convenient available device.

The calibration sample 302 results from this method.

An example of calibration mixture 308 is provided in the following table:

| Calibration compounds | $T_{boiling}$ (°C) | $P_{saturated}$ vapor (kPa @20 °C) | Volume (cc) |
|---|---|---|---|
| iC5 | 28 | 76.1 | 0.9 |
| nC5 | 36.1 | 53.3 | 0.7 |
| 2,2-DMC4 | 49.7 | 36.9 | 0.4 |
| 2-MC5 (2-methylpentane) | 60.3 | | 0.4 |
| nC6 | 68.7 | 20.1 | 0.4 |
| 2,4-DMC5 | 80.6 | | 0.4 |

More generally, the content in iC5 is between 20% and 40% of the volume of the calibration mixture, the content in nC5 is between 20% and 40% of the volume of the calibration mixture, the content in 2,2-DMC4 is between 5% and 15% of the volume of the calibration mixture, the content in nC6 is between 5% and 15% of the volume of the calibration mixture, the content in 2-MC5 is between 5% and 15% of the volume of the calibration mixture, and the content in 2,4-DMC5 is between 5% and 15% of the volume of the calibration mixture.

The application of a process of determining the content of plurality of compounds in a drilling fluid according to the invention will now be described.

This method comprises an initial step for evaluating the correction factors $\rho_1(i)$ of a first group of compounds i to be analyzed, a step for adjusting a model linking the correction coefficients of each compound according to one of their thermodynamic characteristics, a step for calculating from the thereby determined model, correction factors $\rho_2(i)$ of a second group of constituents to be analyzed, and then an online analysis step of the gas content of the drilling fluid circulating in the circulation conduit 25.

The first step for evaluating the correction factors is advantageously carried out by a method of calibration as described in patent application EP-A-1 710 575 of the Applicant, notably in the calibration assembly 20 described in Fig. 2.

For this purpose, the calibration mud 302 is introduced into the upstream tank 111.

The calibration mud 302 is an artificial mud sample realized on the field for the purpose of the extraction efficiency calibration (EEC) of the process of determining the content of plurality of compounds in a drilling fluid.

The calibration mud 302 contains a plurality of first compounds, advantageously among those intended to be analyzed in the drilling fluid circulating in the drilling conduit 25.

The sampling head 61 is then immersed in the upstream tank 111 in order to pump the calibration sample through the pump 65 and the admission conduit 67 as far as the enclosure 71 at a flow rate $Q_m$.

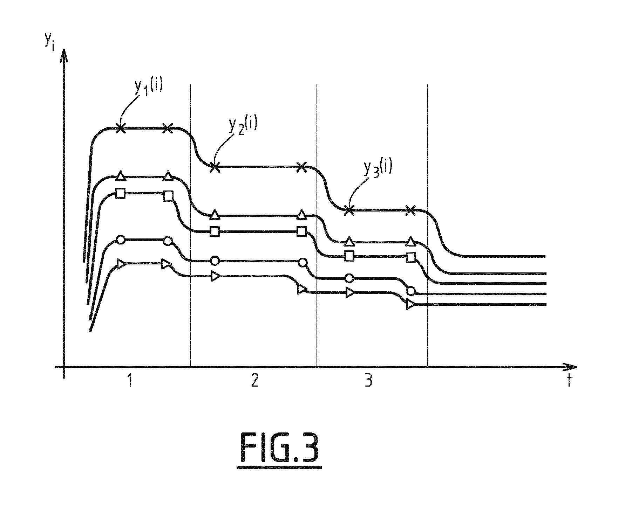

Next, the stirrer 73 having been activated, a gas fraction $y_1(i)$ of each first compound to be measured contained in the calibration mud is extracted and conveyed via the carrier gas introduced through the inlet 77 across the transport line 91 as far as the instrumentation 99. Each gas fraction $y_1(i)$ is then quantified for each compound, as illustrated by Fig. 3.

Next, when the calibration mud has substantially totally passed through the enclosure 71 and been recovered in the tank 87, the tanks 87 and 111 are inverted so that the same calibration mud under the given extraction conditions again passes through the extraction device 53.

A gas fraction $y_2(i)$ of each compound to be analyzed present in the calibration mud is then extracted during this extraction phase.

Next, this operation is repeated for n successive extraction stages, with n being a total number of extraction stages of the same calibration mud advantageously comprised between 2 and 10 as illustrated in Fig. 3.

The computing unit 101 then determines, for each first compound, the definition of a series illustrated on a logarithmic scale by a linear curve from at least two pairs of values $(n, y_n)$ which correspond to the extraction stage n of the gases of the calibration mud and to the given amount $y_n(i)$ of a gas fraction of a compound during the extraction stage n.

This series depends on the gas fraction $y_1(i)$ extracted during a first extraction stage and on a parameter $m(i)$ independent of the extraction stage and characteristic of the compound extracted from the drilling fluid, and of the extraction conditions.

Advantageously, the series determined by the computing unit is substantially an exponential geometrical series which is described by the formula:

$$ y_n(i) = y_1(i) \times \exp\left[-m(i) \times (n-1)\right] $$

Next, a first correction factor $\rho_1(i)$ is calculated for linking the content $t_0(i)$ of each first compound in the drilling fluid to the gas fraction $y_1(i)$ extracted at a total volume flow rate of extracted gases $Q_g$, during a first passage of the fluid in the extraction device 53 and at a volume flow rate $Q_m$, by the equation:

$$ t_0(i) = \frac{Q_g}{Q_m} \cdot \rho(i) \cdot y_i(i) \quad (1) $$

This correction factor $\rho_1(i)$ is then determined by the equation (2) below:

$$ \rho_i(i) = \frac{1}{y_1(i)} \sum_{1}^{\infty} y_n(i) = \frac{1}{1 - \exp(-m(i))} = \frac{1}{1 - \lambda} \quad (2) $$

In one alternative, the correction factors $\rho_1(i)$ of the first group of first compounds are determined by other equations, or even empirically.

Next, the step for calculating the correction factors $\rho_2(i)$ of a second group of compounds to be analyzed is applied.

In a first alternative embodiment of the method, this second group advantageously comprises the heaviest compounds, for example $C_5$-$C_{10}$ hydrocarbons for which the accuracy of the measurement of the extracted gas fractions is lower.

For this purpose, each second correction factor $\rho_2(i)$ is advantageously calculated from a calculation equation posed on the basis of a coefficient $\alpha(i)$ representative of the degassing kinetics of each second compound in the extraction device 53 under the given extraction conditions, and of a coefficient $K(i)$ representative of thermodynamic equilibrium between the gas fraction and the liquid fraction of each second compound present in the extractor 71 of the extraction device 53.

The equation for calculating each second correction factor $\rho_2(i)$ further depends on the volume flow rate $Q_m$ of mud circulating in the enclosure 71, on the average volume $V_g$ of the upper portion 83 forming the gas head space, on the average volume $V_m$ of the lower portion 81 containing the circulating fluid and on the total gas flow rate $Q_g$ sampled through the outlet 79 under the given extraction conditions.

Advantageously, each second correction factor $\rho_2(i)$ is calculated by the equation:

$$ \rho_2(i) = 1 + \frac{Q_m}{V_g} \cdot \frac{1}{\alpha(i) \cdot K(i)} + \frac{Q_m}{V_m} \cdot \frac{1}{\alpha(i)} + \frac{Q_m}{Q_g} \cdot \frac{1}{K(i)} \quad (3) $$

According to the invention, the coefficients $K(i)$ and $\alpha(i)$ are calculated from a characteristic thermodynamic factor Fi specific to each second compound which depends at least on one thermodynamic parameter representative of the second compound, and are also calculated from a plurality of parameters a, b, c, d which are independent of the second compound and of the extraction conditions and which are calculated from each first correction factor $\rho_1(i)$ and from the calculation equation (3) as this will be seen below.

Advantageously, said or each representative thermodynamic parameter is selected from the boiling temperature $\ominus_b(i)$ at atmospheric pressure of the second compound I, from its critical temperature $\ominus_c(i)$ and its critical pressure $P_c(i)$. Said or each characteristic its critical temperature $\ominus_c(i)$ and its critical pressure $P_c(i)$. Said or each characteristic thermodynamic factor is advantageously selected as proposed by *Hoffman (*Hoffman et al. « Equilibrium Constants for a Gas Condensate System » Trans. AIME (1953) 198,1-10*)* or in an improved way by Standing (Standing, « A set of Equations for Computing Equilibrium Ratios of a Crude Oil/Natural Gas System at Pressures below 1,000 psia » SPE 7903 1979).

The characteristic thermodynamic factor Fi is then further calculated according to the temperature ⊖ of the drilling fluid in the enclosure 71 under the given extraction conditions.

Advantageously, the parameter Fi is obtained from an equation linking all the aforementioned parameters such as the following equations:

$$F_i = \frac{\left[\dfrac{1}{\theta_b(i)} - \dfrac{1}{\theta}\right]}{\left[\dfrac{1}{\theta_b(i)} - \dfrac{1}{\theta_c(i)}\right]} \cdot \log\left(\frac{P_c(i)}{P_{atm}}\right) \quad (4)$$

The coefficients K(i) and α(i) are then given by the following equations:

$$K(i) = a \times \exp(b \cdot F_i) \quad (5)$$

$$\alpha(i) = c \times \exp(d \cdot F_i) \quad (6)$$

Thus, the equation (3) above may be re-written for each second compound in the following form:

$$\rho_2(i) = 1 + \frac{Q_m}{V_g} \cdot \frac{1}{a \cdot c \times \exp[(b+d) \cdot F_i]} + \frac{Q_m}{V_m} \cdot \frac{1}{c \times \exp(d \cdot F_i)} + \frac{Q_m}{Q_g} \cdot \frac{1}{a \times \exp(b \cdot F_i)} \quad (7),$$

wherein each second correction factor $\rho_2(i)$ depends on the plurality of parameters a, b, c, d independent of the second compound, determined on the basis of each first correction factor $\rho_1(i)$, and also depends on the characteristic thermodynamic factor Fi of each second compound as defined above, as well as on the volume flow rate Qm of drilling fluid passing through the enclosure 71, on the volume Vg of the upper portion 83 of the enclosure comprising a gas head space, on the average volume $V_m$ of drilling fluid present in the enclosure and on the volume flow rate $Q_g$ of gas extracted from the enclosure.

In order to determine the parameters a, b, c, d, a system of equations is laid out by applying the calculation equation (7) above to each first correction factor $\rho_1(i)$ depending on the thermodynamic parameter Fi of each first compound, according to the system:

$$\rho_1(i) = 1 + \frac{Q_m}{V_g} \cdot \frac{1}{a \cdot c \times \exp[(b+d) \cdot F_i]} + \frac{Q_m}{V_m} \cdot \frac{1}{c \times \exp(d \cdot F_i)} + \frac{Q_m}{Q_g} \cdot \frac{1}{a \times \exp(b \cdot F_i)} \quad (8)$$

This system is solved by an optimization method for example using the least squares technique for obtaining the parameters a, b, c and d independently of each second compound.

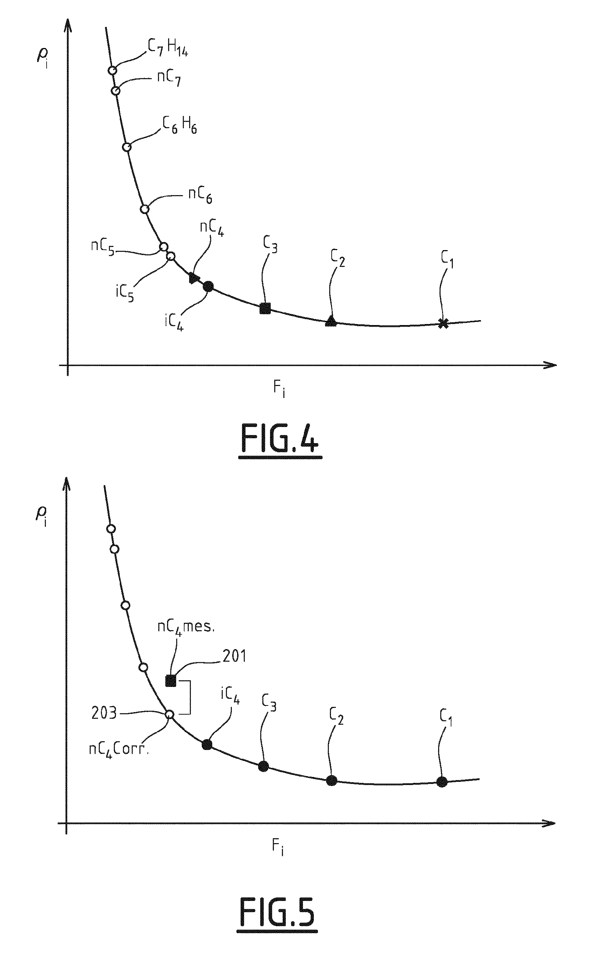

With reference to Fig. 5, once the parameters a, b, c, d are obtained from each first correction factor $\rho_1(i)$ represented in solid symbols in Fig. 5, each second correction factor $\rho_2(i)$ relating to each second compound, represented by hollow symbols in Fig. 5, is calculated by using equation (7) and by calculating for each second compound the coefficient Fi by equation (4).

With the method according to the invention it is therefore possible to obtain all the correction factors of the compounds to be analyzed by a simple calculation based on a not very large number of correction factors determined experimentally or empirically.

This considerably increases the accuracy of the measurement, notably for relatively heavy hydrocarbon compounds.

In one alternative, the whole of the correction factors for each compound to be analyzed, including the first compound, is recalculated from the calculation equation (7).

The analysis step is then applied during the drilling. In order to carry out the drilling, the drilling tool 15 is driven into rotation by the surface installation 41. The drilling fluid is introduced into the inner space 35 of the drilling lining 29 through the injection means 43. This fluid flows down to the drilling head 27 and passes in the drilling conduit 13 through the drilling head 27. This fluid cools and lubricates the piercing means 33. The fluid collects solid debris resulting from the drilling and moves up through the annular space defined between the drill string 29 and the walls of the drilling conduit 13, and is then discharged through the circulation conduit 25.

In this step, the sampling head 61 is positioned in the circulation conduit 25, downstream from the vibrating sieve 45. The pump 65 is then actuated in order to pick up drilling fluid in the conduit 25 with the given volume flow rate $Q_m$ and to introduce it into the enclosure 71 through the admission conduit 67. The drilling fluid then contains the components to be analyzed.

The stirrer 73 is actuated for stirring the drilling fluid present in the lower portion 81 and for extracting a gas fraction $y_1(i)$ of each compound i present in the drilling fluid. This gas fraction $y_1(i)$ is conveyed as far as the instrumentation 99 through the transport line 91 in order to determine its value.

During the extraction, the temperature of the drilling fluid in the enclosure 71, the pressure P of the gas head space located above the fluid present in the enclosure 71, the flow rate $Q_m$ of drilling fluid admitted into the enclosure 71, and the sampled gas flow rate $Q_g$, the nature of the stirring as well as the stirring rate are substantially identical as compared with the same parameters used during the calibration step.

Next, the computing unit 101 infers therefrom the value of the content of each compound i in the drilling fluid by equation (1), where the correction factors $p(i)$ of at least one second group of compounds are calculated with the equation (7) above.

In an alternative application of the method, illustrated in Fig. 5, a plurality of first correction factors $\rho_1(i)$, illustrated in solid symbols in the figure are determined experimentally or empirically.

However, at least one first correction factor determined experimentally or empirically is not taken into account for carrying out the determination of the parameters a, b, c, d.

This correction factor is then excluded and replaced with a correction factor 203 calculated with equation (7).

The method according to the invention thereby allows correction of doubtful or erroneous experimental measurements for example because of contaminants present in the calibration mud.

In one alternative, the second compounds are identical with the first compounds, all the correction factors $\rho_1(i)$ being replaced with optimized correction factors.

In one alternative, the coefficient Fi is equal to the boiling temperature $\ominus_b(i)$ at atmospheric pressure of the compound.

In an alternative embodiment, it is possible to improve the determination of the correction factors $\rho_1(i)$ by only using two successive stages for extracting the calibration mud. In this case, the correction coefficients $\rho_1(i)$ obtained with equation (2) are indeed very sensitive to measurement errors, the exponential decrease coefficient $m(i)$ of equation (2) is no longer obtained via a linear regression but by directly calculating a straight line passing through two points. The calculation of the parameters a, b, c, and d by solving the system of equations (8) and the calculation of optimized correction coefficients for each first compound, as described above, allows these measurement errors to be reduced by introducing an overdimensioned system of equations.

With the method according to the invention it is further possible to improve the calibration method described in Patent Application EP-A-1 710 575 of the Applicant.

For this purpose, the parameters a, b, c and d independent of each compound and extraction conditions are determined in the step for fitting the model as described earlier, by using the system of equations (8) in which the representative parameters of the extraction conditions, $\ominus$, $Q_m$, $Q_g$, $V_m$ and Vg of each calculation equation are those which prevail under the first extraction conditions.

Next, once the coefficients a, b, c, d have been determined, the correction coefficients $p(i)$ for each compound are recalculated with equations (4) and (7) from the new values of the parameters representative of the extraction conditions $\ominus$, $Q_m$, $Q_g$, $V_m$ and Vg under the second extraction conditions.

The computing unit 101 may further take into account any change in these representative parameters of extraction conditions during the analysis step by adjusting in real time the correction coefficients for each measured compound from the new values of $\ominus$, $Q_m$, $Q_g$, $V_m$ and Vg.

## Claims

1. Method of calibration intended to be used in a process of determining the content ($t_0(i)$) of a plurality of compounds contained in a drilling fluid, the method comprising:

- obtaining a calibration mud (302) containing calibration compounds (306);
- performing a gas extraction from said calibration mud (302) with at least two extraction stages in order to obtain at least two extracted gases containing said calibration compounds (306);
- measuring parameters (y1, y2) representative of the quantities (q) of said calibration compounds (306) in said extracted gases; and
- calculating correction factors ($\rho_1(i)$) using said measured parameters (y1, y2), the correction factors ($\rho_1(i)$) being intended to be used in said process in order to calculate said content ($t_0(i)$);
**characterized in that** the calibration mud (302) is an artificial mud sample comprising a sample (304) of a drilling fluid mixed with the calibration compounds (306), the calibration compounds (306) comprising at least two, preferably four, different hydrocarbon compounds being in part in liquid state at operative pressure, temperature and volume conditions.

2. Method according to claim 1, wherein the operative pressure, temperature and volume conditions are atmospheric conditions, and said hydrocarbon compounds (306) having at least 5 carbon atoms.

3. Method according to claim 1 or 2, **characterized in that** each of said hydrocarbon compounds has between 5 and 7 carbon atoms.

4. Method according to claim 3, **characterized in that** each of said hydrocarbon compounds has 5 or 6 carbon atoms.

5. Method according to any of claims 1 to 4, **characterized in that** said calibration compounds (306) are normal pentane, isopentane, normal hexane and 2,2-dimethylpentane

6. Method according to any of claims 1 to 5, wherein each of said calibration compounds (306) has a respective concentration in the calibration mud (302), said concentrations of the calibration compounds being between 0.01 and 1 g/l of mud.

7. Method according to any of claims 1 to 6, wherein said calibration mud (302) is substantially free of surfactant.

8. Process of determining the content (t0) of a plurality of compounds contained in a drilling fluid comprising:

   - implementing the method of any of the preceding claims; and
   - obtaining at least one second sample of the drilling fluid;
   - performing a gas extraction from said second sample in order to obtain at least a third extracted gas containing said plurality of compounds;
   - measuring second parameters (y1(i)) representative of the quantities (q(i)) of said plurality of compounds in said third extracted gas; and
   - calculating said content ($t_0(i)$) using said second parameters and said correction factors ($\rho_1(i)$).

9. Process according to the preceding claim, wherein the step of obtaining at least one second sample of the drilling fluid is iterated.

10. Method of preparation (300) of a calibration mud (302) intended to be used in the method of any of claims 1 to 7, comprising the following steps advantageously performed on a rig:

    - obtaining (A) an initial mud sample (304) from a drilling fluid;
    - obtaining said calibration compounds (306) and mixing (B) them in order to obtain a calibration mixture (308);
    - adding (C) said calibration mixture (308) into said initial mud sample (304) in order to obtain an emulsified mud (310); and
    - obtaining (D) the calibration mud (302) from the emulsified mud (310).

11. Method (300) according to claim 10, wherein the step (C) of adding said calibration mixture (308) into said initial mud sample (304) includes an emulsification (C2) of the calibration mixture (308) and at least a fraction (312) of the initial mud sample (304).

12. Method (300) according to claim 11, wherein said emulsification (C2) is carried out using at least one ultrasonic probe in order to send an ultrasonic signal into said fraction (312) of the initial mud sample.

**13.** Method (300) according to claim 11 or 12, wherein the step (C) of adding said calibration mixture (308) into said initial mud sample (304) comprises the following substeps:

- extracting (C1) mud from the initial mud sample (304) in order to obtain a sub-sample of mud (312) and a remaining part of mud (314);
- emulsification (C2) of the calibration mixture (308) and the sub-sample of mud (312) in order to obtain an emulsified sub-sample (316); and
- mixing (C3) the emulsified sub-sample (316) and the remaining part of mud (314) in order to obtain the emulsified mud (310).

**14.** Method (300) according to claim 13 and claim 12, wherein said ultrasonic signal corresponds to a power comprised between 0.5 and 2.0 W/cm$^3$ of said sub-sample, preferably between 0.75 and 1.5 W/cm$^3$ of said sub-sample.

**15.** Method (300) according to any of claim 12 to 14 in combination with claim 12, wherein said ultrasonic signal has a frequency ranging from 18000 Hz to 25000 Hz, preferably about 20000 Hz.

**16.** Method (300) according to any of claims 10 to 15, wherein said step (D) of obtaining the calibration mud includes homogenizing said emulsified mud (310) in order to obtain said calibration mud (302).

**Amended claims in accordance with Rule 137(2) EPC.**

**1.** Method of calibration Intended to be used in a process of determining the content ($t_0(l)$) of a plurality of compounds contained in a drilling fluid, the method comprising:

- obtaining a calibration mud (302) containing calibration compounds (306);
- performing a gas extraction from said calibration mud (302) with at least two extraction stages in order to obtain at least two extracted gases containing said calibration compounds (306);
- measuring parameters (y1, y2) representative of the quantities (q) of said calibration compounds (306) in said extracted gases; and
- calculating correction factors ($\rho_1(i)$) using said measured parameters (y1, y2), the correction factors ($\rho_1(l)$) being intended to be used in said process in order to calculate said content ($t_0(i)$);

**characterized in that** the calibration mud (302) Is an artificial mud sample comprising a sample (304) of a drilling fluid mixed with the calibration compounds (306), the calibration compounds (306) comprising at least two, preferably four, different hydrocarbon compounds being In part in liquid state at operative pressure, temperature and volume conditions.

**2.** Method according to claim 1, wherein the operative pressure, temperature and volume conditions are atmospheric conditions, and said hydrocarbon compounds (306) having at least 5 carbon atoms.

**3.** Method according to claim 1 or 2, **characterized in that** each of said hydrocarbon compounds has between 5 and 7 carbon atoms.

**4.** Method according to claim 3, **characterized in that** each of said hydrocarbon compounds has 5 or 6 carbon atoms.

**5.** Method according to any of claims 1 to 4, **characterized in that** said calibration compounds (306) are normal pentane, isopentane, normal hexane and 2,2-dimethylpentane

**6.** Method according to any of claims 1 to 5, wherein each of said calibration compounds (306) has a respective concentration in the calibration mud (302), said concentrations of the calibration compounds being between 0.01 and 1 g/l of mud.

**7.** Method according to any of claims 1 to 6, wherein said calibration mud (302) is substantially free of surfactant.

**8.** Process of determining the content (t0) of a plurality of compounds contained in a drilling fluid comprising:

- implementing the method of any of the preceding claims; and

- obtaining at least one second sample of the drilling fluid;
- performing a gas extraction from said second sample in order to obtain at least a third extracted gas containing said plurality of compounds;
- measuring second parameters (y1(i)) representative of the quantities (q(i)) of said plurality of compounds in said third extracted gas; and
- calculating said content ($t_0(i)$) using said second parameters and said correction factors ($\rho_1(i)$).

**9.** Process according to the preceding claim, wherein the step of obtaining at least one second sample of the drilling fluid is iterated.

**10.** Method of preparation (300) of a calibration mud (302) used in the method of any of claims 1 to 7, comprising the following steps advantageously performed on a rig:

- obtaining (A) an initial mud sample (304) from a drilling fluid;
- obtaining said calibration compounds (306) and mixing (B) them in order to obtain a calibration mixture (308);
- adding (C) said calibration mixture (308) into said initial mud sample (304) In order to obtain an emulsified mud (310); and
- obtaining (D) the calibration mud (302) from the emulsified mud (310).

**11.** Method (300) according to claim 10, wherein the step (C) of adding said calibration mixture (308) into said initial mud sample (304) includes an emulsification (C2) of the calibration mixture (308) and at least a fraction (312) of the initial mud sample (304).

**12.** Method (300) according to claim 11, wherein said emulsification (C2) Is carried out using at least one ultrasonic probe In order to send an ultrasonic signal into said fraction (312) of the initial mud sample.

**13.** Method (300) according to claim 11 or 12, wherein the step (C) of adding said calibration mixture (308) into said initial mud sample (304) comprises the following substeps:

- extracting (C1) mud from the initial mud sample (304) In order to obtain a sub-sample of mud (312) and a remaining part of mud (314);
- emulsification (C2) of the calibration mixture (308) and the sub-sample of mud (312) In order to obtain an emulsified sub-sample (316); and
- mixing (C3) the emulsified sub-sample (316) and the remaining part of mud (314) In order to obtain the emulsified mud (310).

**14.** Method (300) according to claim 13 and claim 12, wherein said ultrasonic signal corresponds to a power comprised between 0.5 and 2.0 W/cm$^3$ of said sub-sample, preferably between 0.75 and 1.5 W/cm$^3$ of said sub-sample.

**15.** Method (300) according to any of claim 12 to 14 in combination with claim 12, wherein said ultrasonic signal has a frequency ranging from 18000 Hz to 25000 Hz, preferably about 20000 Hz.

**16.** Method (300) according to any of claims 10 to 15, wherein said step (D) of obtaining the calibration mud includes homogenizing said emulsified mud (310) In order to obtain said calibration mud (302).

FIG.1

EP 2 703 597 A1

FIG.2

FIG.3

$\rho_i$

$C_7 H_{14}$

$nC_7$

$C_6 H_6$

$nC_6$

$nC_5$

$nC_4$

$iC_5$

$iC_4$

$C_3$

$C_2$

$C_1$

$F_i$

## FIG.4

$\rho_i$

$nC_4 mes.$

201

203

$iC_4$

$C_3$

$C_2$

$C_1$

$nC_4 Corr.$

$F_i$

## FIG.5

**FIG.6**

Europäisches
Patentamt

European
Patent Office

Office européen
des brevets

# EUROPEAN SEARCH REPORT

Application Number

EP 12 30 6047

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/303463 A1 (LESSI JACQUES [FR]) 15 December 2011 (2011-12-15) | 1-7 | INV.<br>E21B21/06<br>G01N33/28 |
| Y | * paragraphs [0003], [0019], [0047] - [0049]; figure 2 * | 10-16 | |
| A | | 8,9 | |
| X | US 2006/224333 A1 (FRECHIN NICOLAS [FR] ET AL) 5 October 2006 (2006-10-05) | 1-9 | |
| Y | * figures 2-5 * | 10-16 | |
| Y | US 5 161 409 A (HUGHES TREVOR [GB] ET AL) 10 November 1992 (1992-11-10)<br>* column 12 - paragraph calibration; figures 3-7 * | 10-16 | |
| A | US 6 443 001 B1 (DURIEZ GILBERT [FR] ET AL) 3 September 2002 (2002-09-03)<br>* the whole document * | 1-16 | |
| A | US 4 635 735 A (CROWNOVER DONALD J [US]) 13 January 1987 (1987-01-13)<br>* the whole document * | 1-16 | TECHNICAL FIELDS SEARCHED (IPC)<br><br>E21B<br>G01N |

The present search report has been drawn up for all claims

| Place of search | Date of completion of the search | Examiner |
|---|---|---|
| Munich | 6 February 2013 | Klein, Marc-Oliver |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**           EP 12 30 6047

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2013

| Patent document cited in search report | | Publication date | Patent family member(s) | | | Publication date |
|---|---|---|---|---|---|---|
| US 2011303463 | A1 | 15-12-2011 | AU | 2010205535 | A1 | 08-09-2011 |
| | | | CA | 2749387 | A1 | 22-07-2010 |
| | | | EP | 2380017 | A2 | 26-10-2011 |
| | | | FR | 2941261 | A1 | 23-07-2010 |
| | | | US | 2011303463 | A1 | 15-12-2011 |
| | | | WO | 2010081981 | A2 | 22-07-2010 |
| US 2006224333 | A1 | 05-10-2006 | AT | 491154 | T | 15-12-2010 |
| | | | CA | 2541623 | A1 | 04-10-2006 |
| | | | EP | 1710575 | A2 | 11-10-2006 |
| | | | FR | 2883916 | A1 | 06-10-2006 |
| | | | NO | 327508 | B1 | 27-07-2009 |
| | | | US | 2006224333 | A1 | 05-10-2006 |
| US 5161409 | A | 10-11-1992 | CA | 2028053 | A1 | 29-04-1991 |
| | | | EP | 0426231 | A2 | 08-05-1991 |
| | | | GB | 2237305 | A | 01-05-1991 |
| | | | NO | 904659 | A | 29-04-1991 |
| | | | US | 5161409 | A | 10-11-1992 |
| US 6443001 | B1 | 03-09-2002 | AT | 382146 | T | 15-01-2008 |
| | | | AU | 7429700 | A | 24-04-2001 |
| | | | BR | 0007171 | A | 07-08-2001 |
| | | | CA | 2352103 | A1 | 29-03-2001 |
| | | | DE | 60037570 | T2 | 08-01-2009 |
| | | | DK | 1131615 | T3 | 13-05-2008 |
| | | | EP | 1131615 | A1 | 12-09-2001 |
| | | | ES | 2298154 | T3 | 16-05-2008 |
| | | | FR | 2799790 | A1 | 20-04-2001 |
| | | | NO | 20012536 | A | 25-05-2001 |
| | | | US | 6443001 | B1 | 03-09-2002 |
| | | | WO | 0122050 | A1 | 29-03-2001 |
| US 4635735 | A | 13-01-1987 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2799790
- EP 1710575 A
- EP 2380017 A
- EP 1887343 A

**Non-patent literature cited in the description**

- **HOFFMAN et al.** Equilibrium Constants for a Gas Condensate System. *Trans. AIME,* 1953, vol. 198, 1-10
- **STANDING.** A set of Equations for Computing Equilibrium Ratios of a Crude Oil/Natural Gas System at Pressures below 1,000 psia. *SPE 7903,* 1979